Europäisches Patentamt

(19) European Patent Office

Office européen des brevets

(11) Publication number: **0 102 816**
**A2**

# EUROPEAN PATENT APPLICATION

(12)

(21) Application number: 83304939.8

(22) Date of filing: 25.08.83

(51) Int. Cl.³: **A 61 B 5/02**
**A 61 B 5/00, G 01 N 21/25**

(30) Priority: 02.09.82 US 414174
13.09.82 US 417311

(43) Date of publication of application:
14.03.84 Bulletin 84/11

(84) Designated Contracting States:
AT BE CH DE FR GB IT LI LU NL SE

(71) Applicant: NELLCOR INCORPORATED
3116 Diablo Avenue
Hayward California(US)

(72) Inventor: New, William, Jr.
95 Skywood Way
Woodside California(US)

(72) Inventor: Corenman, James E.
708 Harbor Road
Alameda California(US)

(74) Representative: Allen, Oliver John Richard et al,
Lloyd Wise, Tregear & Co. Norman House 105-109
Strand
London, WC2R 0AE(GB)

(54) **Pulse oximeter.**

(57) A pulse oximeter is disclosed of the type wherein light of two different wavelengths is passed through human or animal body tissue, such as a finger, an ear, the nasal septum or the scalp, so as to be modulated by the pulsatile component of arterial blood therein, and thereby allowing indication of oxygen saturation. The level of incident light is continually adjusted for optimal detection of the pulsatile component, while permitting accommodation to variable attenuations due to skin color, flesh thickness and other invariants. At significant slope reversal of the pulsatile component to negative (indicating a wave maximum), wave form analysis of blood flow occurs. A quotient of the pulsatile component of light transmission over the constant component of light transmission is measured for each of two wave lengths by direct digital tracking. The respective quotients are thereafter converted to a ratio, which ratio may be thereafter fitted to a curve of independently derived of oxygen saturation. Calibration is disclosed by solving four unknowns at at least four differing saturations. An output of pulse rate, pulse flow and oxygen saturation is given. An incident light source duty cycle is chosen to be at least 1 in 4 so that noise, inevitably present in the signal, may be substantially eliminated and filtered. Provision is made for a local battery-driven low power consumption instrument capable of being substantially independent of ambient light noise.

EP 0 102 816 A2

## PULSE OXIMETER

This invention relates to pulse oximeters.

A serious problem exists in operating rooms. Specifically, the chemical determination of oxygen level in blood consumes at least 3 to 5 minutes. A patient deprived of blood oxygen for such a duration typically incurs irreversible brain damage if not death. Conventional and commonly used indications of life, such as pulse, blood pressure and electrocardiograms, do not always provide a sufficient warning of respiratory and circulatory catastrophes. For example, in sudden respiratory arrest, heart rate can remain within normal limits until well after irreversible brain damage occurs.

Electronic, non-invasive techniques for determination of oxygen content are known. U.S. Patent 2,706,927 to Wood disclosed the computation of oxygen saturation from measurement of light absorption of body tissue at two wavelengths. A "bloodless" measurement was first taken in which as much blood as possible was squeezed from the area where measurement was taken. Thereafter, arterial blood was allowed to flow into the tissue as the condition of normal blood flow was restored. A comparison of the light absorption in the two states provided information on the arterial oxygen saturation of the subject. A series of devices and procedures have been founded using this technology.

2

In procedures based on this technology, difficulty has been experienced in reliably determining the "bloodless" parameters due in part to geometrical distortion due to the compression of the tissue; imperfect measurement of this parameter gave imperfect results.

The transmission of light of each wavelength is a function of the thickness, color, and structure of skin, flesh, bone, blood and other material through which the light passes. This attenuation in transmission has been asserted to have a logarithmic characteristic, in accordance with Lambert-Beers Law.

In a pulse oximeter, the primary material of interest is pulsitile arterial blood. Arterial blood is the only material whose quantity in the tissue varies with time in synchrony with the beating of the heart. Variations in light transmission therefore indicate variations in blood flow permitting direct optical recording of the pulsatile component of arterial blood flow. This ability to separate out the light absorption of arterial blood is especially convenient; since the oxyhemoglobin component of blood is a substance for which the absorption coefficients can be determined, the fraction of oxyhemoglobin in arterial blood can be determined.

Optical plethysmographs are well known. Such instruments measure pulse rate and provide information on the quantity of blood forced into the tissues on each heart beat. These instruments generally utilize a light frequency near or at the isobestic point where measurement of pulsatile flow is made independent of oxygen saturation. Consequently, they intentionally eliminate information on oxygen saturation.

Following the Wood 2,706,927 patent, numerous attempts have been directed at eliminating the difficulties connected with arterial saturation measurements using light absorption. Where the analysis requires the comparing of the "bloodless" measurement either artificially induced or naturally occurring during the rest state of the heart cycle with the measurement of fresh arterial blood when fresh arterial blood enters the tissue. For example, the signal received has been divided into its "AC" and "DC" components and passed through a log amplifier before digital analysis of the signal occurs. See Koneshi et al., U.S. Patent 3,998,550. Likewise, a generation at both wavelengths of subtraction outputs has been utilized before digital analysis. Subtraction outputs have been used to eliminate the DC component and to approximate the logarithmic response of the prior art. See Hamaguri, U.S. Patent 4,266,554. Simply stated, because the pulsatile component constitutes a small portion of the total signal of transmitted light, numerous manipulations based on logarithms have been attempted to screen out the unchanging component of the resultant signal before analysis.

Schemes based on logarithms and logarithm amplifiers have difficulty. These difficulties include the non-reproducability of mass produced log amplifiers. Moreover, noise and semiconductor temperature and voltage sensitivities all serve to limit the practical application of such approximation techniques, utilizing logarithmic amplifiers.

U.S. Patent 3,704,706 to Herczfeld et al disclosed use of a single coherent red light source, preferably a laser. Use of a single light source is unable to separate information dealing with the arterial flow component from that dealing with the arterial oxygen component. The output of such a single red light source instrument can only be an indication of the product of blood flow and the saturation level present. Neither blood flow alone or saturation alone can be known.

Further calibration techniques are simply not set forth and discussed in the prior art. Instruments, mass produced, must be designed for initial calibration. Likewise, maintenance of calibration is not set forth.

A pulse oximeter according to one embodiment of the invention has means wherein light of two different wavelengths is passed through human or animal body tissue, such as a finger, an ear, the nasal septum or the scalp, so as to be modulated by the pulsatile component of arterial blood therein, and thereby allowing indication of oxygen saturation, blood perfusion and heart rate. The leval of incident light is continually adjusted for optimal detection of the pulsatile component, while permitting accommodation to variable attenuations due to skin color, flesh thickness and other invariants. At significant slope reversal in the amplitude of the light transmission of the pulsatile component from negative to positive (indicating a wave maximum), wave form analysis of blood flow occurs. A quotient of the pulsatile component of light transmission over the constant component of light transmission is measured for each of two wavelengths by direct digital tracking. The respective quotients are thereafter converted to a ratio, which ratio may be thereafter fitted to a curve of independently obtained oxygen saturation. Calibration is disclosed by solving four unknowns at at least four differing saturations, or alternatively by fitting the ratio to the independently derived curve and determining at least two coefficients of a polynomial. An output of pulse rate, pulse flow and oxygen saturation is given. An incident light source duty cycle is chosen to be at least 1 in 4 so that noise, inevitably present in the signal, may be substantially eliminated and filtered.

This embodiment uses two light emitting diodes (LEDs) as sources, since they emit relatively narrow band light and are easily packaged. The LEDs are strobed in sequence at a frequency removed as far as practical from ordinary room lights or their harmonic frequencies.  By strobing the LEDs sequentially, a photosensor's resultant signal (representing the inverse of absorption) can be broken into two signals, representing the transmission through the tissue of light at each wavelength.

Each of these two signals is developed by a phase-detection circuit that causes non-strobed ambient light to be canceled on alternate half-cycles.  A subsequent low pass filter removes high frequency noise as well as the modulation (strobing) frequency.

These two signals, representing the transmission of each wavelength through the tissue, are converted to digital values by the microprocessor.  Since the change in transmission caused by the pulsatile variation of blood flow is measured, the instantaneous voltage representing total light transmission must be preserved. Care is exercised to avoid offset voltage, waveform distortion, noise and the like.  Since the pulsatile change is generally only a few percent of the total light transmitted, it is not necessary to digitally convert the entire 10 volt range - rather only the top 25% of the range is converted.  This conversion occurs at the 0-10V analog to digital converter by electronic "magnification" with 4X gain and removal of a fixed 7.5 volt offset.  There results a four times improvement in resolution.

To avoid varying tissue density from causing out-of-range signals, the LED's brightness can be controlled by the microprocessor so that maximum transmission levels received by the photosensor will be within the optimum range. As variations in tissue density occur not only from patient to patient, but also in the same patient as a function of that patient's physiology, LED brightness monitoring and controlling reoccurs continually.

The resultant signals are examined by the microprocessor for the time-varying pulse.  Since the blood flows more quickly than it ebbs, signal maximum
and the change in slope representing increased absorption is used to identify pulse beginning.  Signal
minimum and the change in slope representing maximum
absorption are used to define the end of a pulse.
These values are preferably sensed by the infrared
light signal.  The change in signal from maximum to
minimum represents the absorption of arterial blood,
that is, the absorption caused when new (arterial)
blood flows into the tissues.  The saturation is then
found by analyzing the ratio of transmissions at each
of the wavelengths.  Logarithmic analysis is avoided.

A pulse oximeter is provided according to
one embodiment of the invention in which the pulsatile
component of transmitted light relative to incident
light is directly analyzed.  According to this
arrangement, no logarithmic or analog computation
of the received signal occurs.  Pulse analysis commences
upon increasing light absorption signalled by slope
reversal, the pulse is thereafter digitially tracked
with respect to time.  Analysis occurs only of
pulsatile blood inflow.  During periods when the pulse
is not analyzed, that is, during venous draining of the
analyzed tissue, the level of incident light is
adjusted to bring the signal into the optimum range of
amplitude.

An advantage of the disclosed technique is
that the pulsatile component can be optimized by adjustment of the level of incident light.  The optimization
can occur for substantially all different patients even
though the nonpulsatile component of the received light
varies from patient to patient due to changes of skin,
bone, flesh and veinous blood.  The arterial component
can be adjusted for optimal measurement although it
constitutes but a small fraction of the light signal
transmitted.

A further advantage of this embodiment of the invention is that adjustment of the light level can occur while an individual patient is being monitored. Thus changes in patient physiology which affect either the constant or pulsatile components of light received can be optimized.

Yet another advantage of the disclosed instrument is that the instrument is self-adjusting during operation. For example, the intensity of incident light is adjusted during instrument operation without interference with instrument output.

A further advantage is speed of response to changes and minimization of motion artifacts when compared to prior art AC/DC techniques.

The embodiment of this invention is such that it can simultaneously trace and indicate the pulse amplitude as well as the degree of oxygen saturation of the individual. According to this arrangement, the amplitude of at least one of the wavelengths of light, preferably infrared, is monitored for slope change. A signal is emitted proportional to the rate of occurrence of slope change events to indicate heart rate. A second signal is emitted proportional to the intensities of the light received relative to the incident light at both wavelengths. Visual and audible signals containing pulse rate and oxygen saturation information are emitted.

An advantage of this embodiment of the invention is that each pulsatile component is individually analyzed. The hear beat, arterial oxygen content, and an indication of blood perfusion of the patient are continually monitored.

In an embodiment of this invention the received signal of transmitted light is amplified in the critical range which cancels out ambient light noise. The amplifier first receives a light signal containing the pulsatile light component(plus ambient light noise) for positive amplification. Thereafter, both light sources, there being two in this case, (LEDs) are turned off and the same amplifier receives only light noise which is negatively amplified. This switching effectively subtracts out noise from the pulsatile light component when both signals are sent through a low pass filter where high frequency noise and the modulation frequency are not passed. The remaining signal is amplified over the output range of the amplifier and subjected to a comparison circuit. Comparison by a twelve bit digital to analog converter sampling the pulsatile component in the range of 4096 parts to one part occurs. Precise digital tracking of the resultant signal is enabled.

An advantage of this aspect of the invention is that ambient noise inevitably present in such a signal, can be minimized. For example, ambient room lighting noise can be subtracted out.

In order to ensure the oximeter of this invention is calibrated correctly, the ratio of light reception at two distinct frequencies is determined by the calibrating instrument. At least four coefficients from the earlier determination are initially utilized for at least four separate oxygen saturations these saturations being preferably determined by laboratory techniques. Constant correction factors are thereafter determined by curve fitting the coefficients to the laboratory saturations, these correction factors are thereafter placed into instrument memory. Utilizing these calibrations, electronic readings are attained which duplicate laboratory blood oxygen saturation data.

A method of calibrating an oximeter according to the invention comprises providing an instrument having first and second sources for emitting sequential light pulses in the red and infrared into the flesh of a human; determining a quotient for each said light sources, said quotient including the change of light transmission due to the pulsatile component of blood in the flesh of a human relative to the total light transmission; taking a ratio of said quotients, this ratio being for relation to specific saturation of oxygen in the pulsatile blood of an individual; for at least four said ratios computing at least four differing .saturations by conventional blood oxygen saturation techniques; and curve fitting said ratios to said laboratory saturations to determine constants of curvature whereby for all saturations lying along said curve oxygen content is predictable from said ratios.

An advantage of this embodiment of the invention is that the resultant experimentally produced constants can be reproducibly passed to subsequent instruments. There is no requirements that such instruments be individually calibrated to conventional laboratory blood tests.

A further aspect of this embodiment is to provide apparatus for adjustment of instrument determined light transmission ratio. According to this aspect of the invention, a drive means suitably a motor having a rate of rotation preferably adjustable proportional to the pulse rate to be measured has at least one filter segment attached thereto and rotated by the motor. A detector with two attached LED's and one photosensor is placed to light pipes, preferably fiber optics, so that emitted light and received light pass through the path of the rotating filter. The rotating filter produces an artificial pulse which is used for adjustment of the ratios of light received. The filter is preferably tailored with neutral density filters to emulate the pulsatile shape of a pulse. The filter preferably has a red and infrared component, and preferably the filters in each channel have the same optical properties.

An advantage of this aspect of the invention is that instrument calibration either at the time of manufacture or thereafter (as an adjunct instrument maintenance) is facilitated.

Embodiments of the invention will now be described by way of example with reference to the accompanying drawings in which:-

Fig. 1 is a perspective view of the instrument of this invention illustrating the instrument housing and attachment of a sensor to the digit of a patient;

Fig. 2 is an overall circuit schematic of this invention;

Fig. 3 is a circuit schematic in the vicinity of the microprocessor;

Fig. 4. is a circuit schematic in the vicinity of the read only memory or ROM of this invention;

Fig. 5 is a circuit schematic in the vicinity of the read only memory or RAM of this invention;

Fig. 6 is a circuit schematic of the memory select;

Fig. 7 is a circuit schematic of the input/output select;

Fig. 8 is a circuit schematic of the counter of this invention;

Fig. 9 is a circuit schematic of the comparator circuit wherein 12 bit digital to analog conversion occurs;

Fig. 10 is a circuit schematic of the sample - hold circuity of this invention.

Fig. 11 is a circuit schematic of the offset amplifier circuit of this invention;

Fig. 12 is a circuit schematic of the detector preamp of this invention;

Fig. 13 is a detail of a clock circuit having an output for powering the light emitting diodes;

Fig. 14 is a detail of circuitry for powering the light emitting diodes, the diodes being switched at a point proximate to the detector.

Fig. 15 is a schematic of the apparatus used for adjustment of instrument light ratio detector enabling full adjustment of the instrument at manufacture and later in the field; and

Fig. 16 is a plot of the ratio of light transmission (R) with respect to tested oxygen saturations (S).

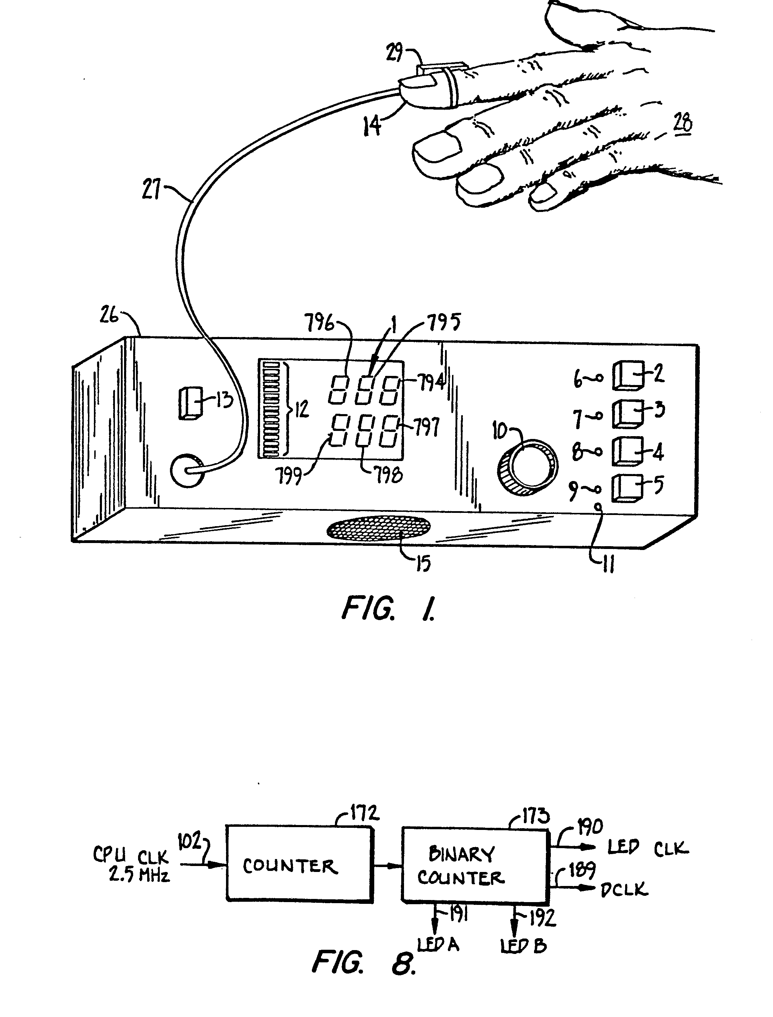

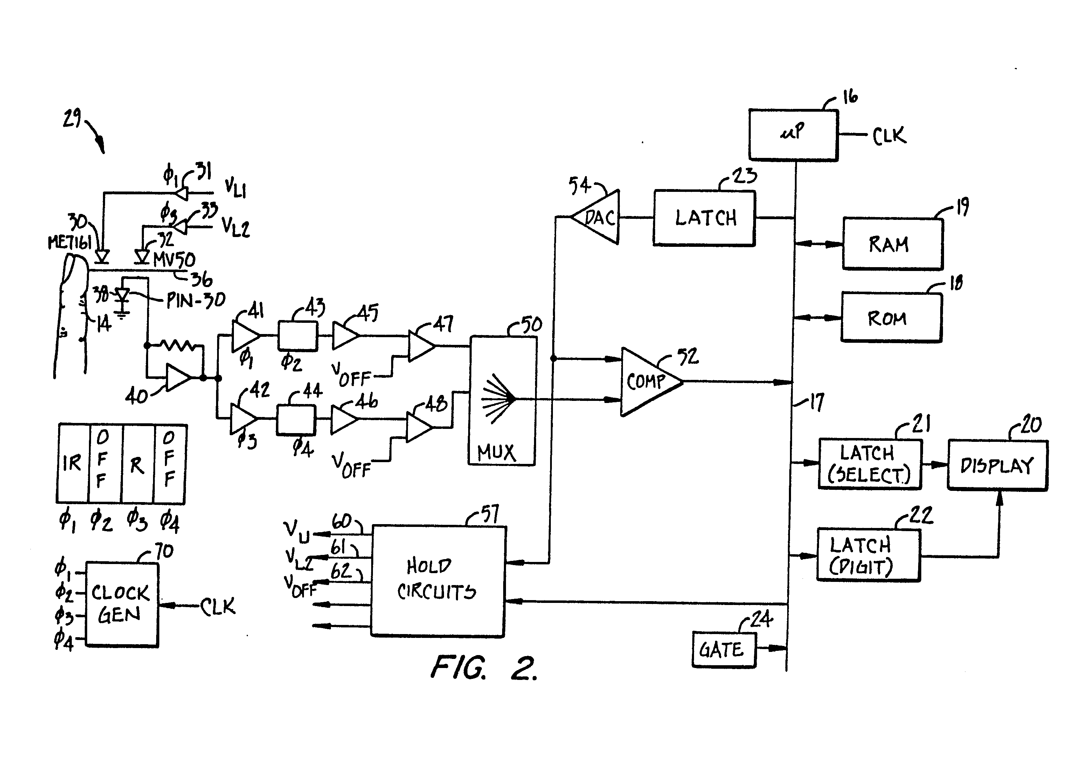

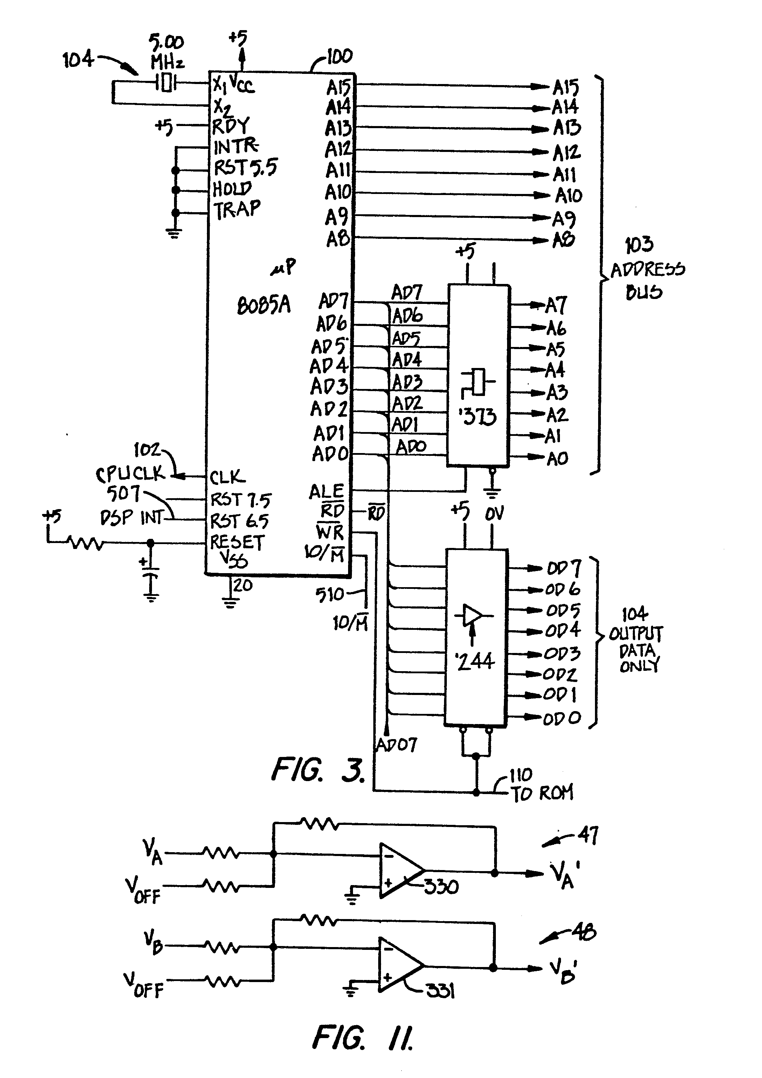

Referring to Fig. 1, the instrument housing 26 of this invention is illustrated. Outwardly, the housing includes a digit display 1, circuitry select button array 2 through 5, alarm status lights 6 through 9, an optically coupled adjustment knob 10, sync status light 11, LED digital viewmeter 12, and power switch 13. A speaker 15 is placed under and in the instrument housing.

From a connector (not shown) in housing 26 there extends leader wires 27. Wires 27 extend to a detector probe 29. Detector 29 is placed upon the finger 14 of a patient 28. Utilizing the placement of the detector 29 at the finger 14, all of the readings in this invention are made possible.

A broader view of the operatability of this invention can be made by considering carefully the circuit schematic of Fig. 2.

Referring to Fig. 2, conventional microprocessor 16 has a bus 17 extending therefrom. Bus 17 has connected thereto conventional ROM 18 and RAM 19. An LED display 20 is schematically illustrated having a select latch 21 and a digit designation latch 22. The circuit select button array 2-5 and optically coupled control knob 10 previously illustrated are gated through controls generally denominated 24.

Having set forth the more or less conventional portions of the microprocessor, attention will now be directed to the analog portions of the circuitry.

Finger 14 of patient 28 is illustrated with detector 29 having schematic detection circuitry. First light emitting diode 32 in the red range and a second light emitting diode 30 in the infrared range are sequentially pulsed to emit light in their respective frequencies by amplifiers 31,33. Typically, LED 32 is in the 6600 angstrom range with LED 30 being in the 9400 angstrom range.

It is necessary that all the light from the active light emitting diode go through the flesh in finger 14. Therefore, a light impervious barrier 36 is placed between photosensor 38 and finger 14. Barrier 36, terminating in contact with the flesh of finger 14, makes the path between the respective light emitting diodes 30, 32 and the light receiving diode 38 occur only through the flesh of finger 14.

In the instrument herein we utilize two discrete frequencies. These frequencies are 670 nanometers (red) and 940 nanometers (infrared). A small amount of discussion related to these parameters is in order.

First, the frequencies are chosen so that they are far enough apart so that the transmission of light appreciably varies with changes in oxygen saturation.

Secondly, the frequencies are chosen so that the same tissue is sampled. For example, a frequency in the ultraviolet would not sample the same tissue due to scattering.

While frequencies extremely close could be used, we have chosen not to do so. We find that drifting of light source frequency can occur with accompanying problems.

Signal received from the respective light emitting diodes first passes through a pre-amplifier 40. This signal is thereafter amplified in parallel at amplifiers 41, 42. As amplified, the signal is passed in parallel from each amplifier through respective phase detectors 43, 44. Passage through respective low pass filters 45, 46 thereafter occurs. Amplification at offset amplifiers 47, 48 then takes place. The pulsatile component is passed to multiplexer 50.

Multiplexer 50 has output to a comparator 52. Comparator 52 is ramped in half steps by a 12 bit digital to analog converter (hereinafter DAC) 54. DAC 54 places a comparison signal divided in one part from 4096 parts with the comparator outputting to bus 17.

The reader will recognize that not all human fingers and appendages are the same. Specifically, the difference between the races, skin pigment, weight, age, maturity and other factors all can lead to different signals being sensed at photosensor 38, even though the frequency and intensity of the light signal output at each of the diodes 30, 32 is the same.

Accordingly, microprocessor 16 is programmed to receive signal from photosensor 38 within an optimum range. Utilizing a second operating phase of DAC 54, and communicating signal to a sample hold 57, the individual LED's 30, 32 are given voltage outputs 60, 61. These voltage outputs 60, 61 are adjusted so that in each case photosensor 38 looks at a signal well withing the range of the DAC.

Clock 70 controls the sequential output of light from the light emitting diodes 30, 32 to a duty cycle of at least 1 in 4. This is schematically illustrated by signals $\emptyset1$ through $\emptyset4$. Reception of signal at detector 43 occurs during time periods $\emptyset1$ and $\emptyset2$ and reception of signal occurs at detector 44 during time periods $\emptyset3$ and $\emptyset4$.

It can be immediately realized that during respective time periods $\emptyset 1$, $\emptyset 3$ active signal from the light emitting diodes 30, 32 is being received. During the time periods $\emptyset 2$ and $\emptyset 4$, no signal and only noise is being received. As will hereinafter become apparent, by amplifying the negative signal before passage through the low pass filter, noise can be subtracted out utilizing the illustrated 1 in 4 duty cycle.

Having given the reader an overview of the circuitry utilized with this invention, the invention will now be discussed in detail.

Referring to Figure 3, the microprocessor 100 is illustrated having an attached crystal 104. This crystal, in combination with clock circuitry incorporated within the microprocessor 100, generate the clock signals required by the microprocessor chip itself as well as providing clock pulses to the rest of the oximeter circuitry through output 102.

Microprocessor 100 is an 8085A CPU integrated circuit chip available from Intel Corporation of Santa Clara, California. The family identification suffixes of the remaining IC components are listed on the drawing and the components are readily available from various other manufacturers.

An address bus 103 includes address lines A0 through A15. To accommodate the eight bit processor, lines A0 through A7 on the address bus are latched from microprocessor pins AD$\emptyset$ through AD7 so that during the address time state these lines may be read. During an alternate time state, lines AD0 through AD7 become output data lines 104, OD$\emptyset$ through OD7, which lines as here configured are only capable of outputting data.

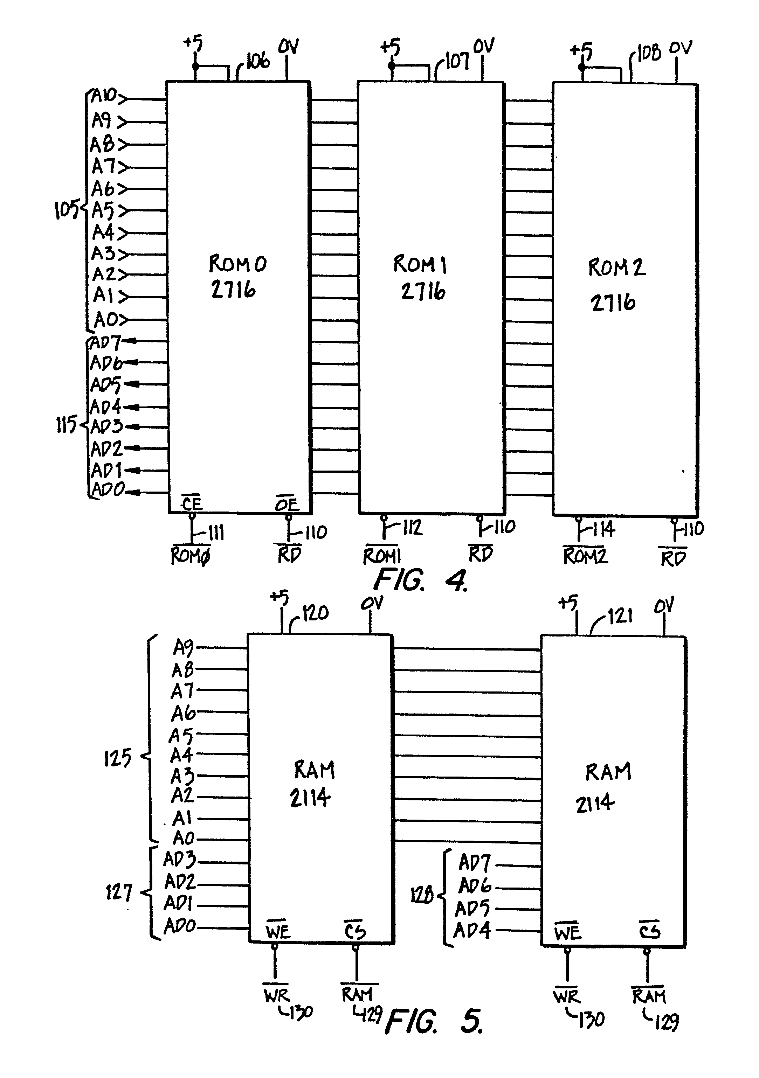

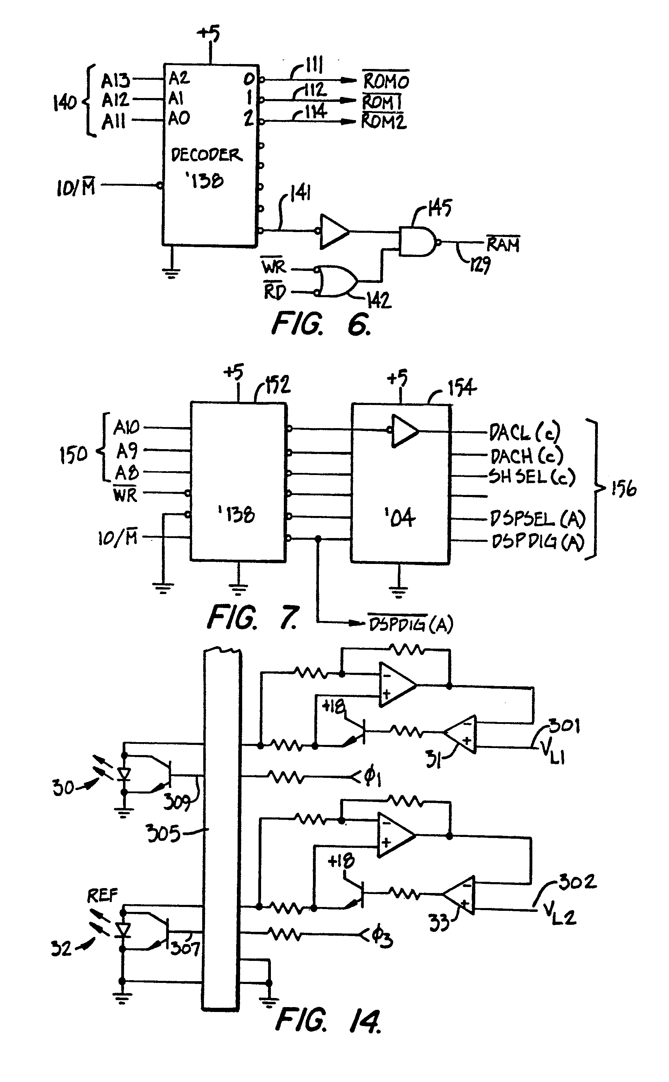

Referring to Fig. 4, the ROM configuration is seen to be standard. The ROM is addressed with a conventional address bus including lines A0 to A10 addressing in parallel ROMs 106, 107 and 108. These respective ROMs are enabled by three decoded address bits from lines A11-A13 (see Fig. 6). As will hereinafter be set forth with respect to Fig. 6, enabling outputs for reading of the ROMs include read enable 110 (see Figs. 3, 4) and specific ROM addresses including ROM 0 address 111, ROM 1 address 112, and ROM 2 address 114. The particular ROMs here utilized are of the · optically erasable programable read only memory variety and include an output data bus 115.

Referring to Fig. 5, two conventional RAMs 120, 121 are shown addressed in parallel at address bits A0 through A9 at bussing 125. These RAMs write and read over eight bits with four bus lines AD0 to AD3 at bus 127 addressing RAM 120 and AD4-AD7 addressing RAM 121 at bussing 128. RAMs 120, 121 are read when enabled through enable ports 129 in the absence of a write signal on port 130. These RAMs are written when enabled by port 129 in the. presence of a write signal through write ports 130. As each of the RAMs connect to four separate data bits, individual enabling of each of the RAMs is not required.

Referring to Fig. 6, the memory select circuit of this invention is illustrated. The memory select has a three bit input 140 at lines A11-A13. Output occurs when memory is selected at ROM 0 enable 111, ROM 1 enable 112, ROM 2 enable 114. A RAM enable 141 passes through an inverter and NAND gate to enable reading of RAMs 120, 121 for either reading or writing.

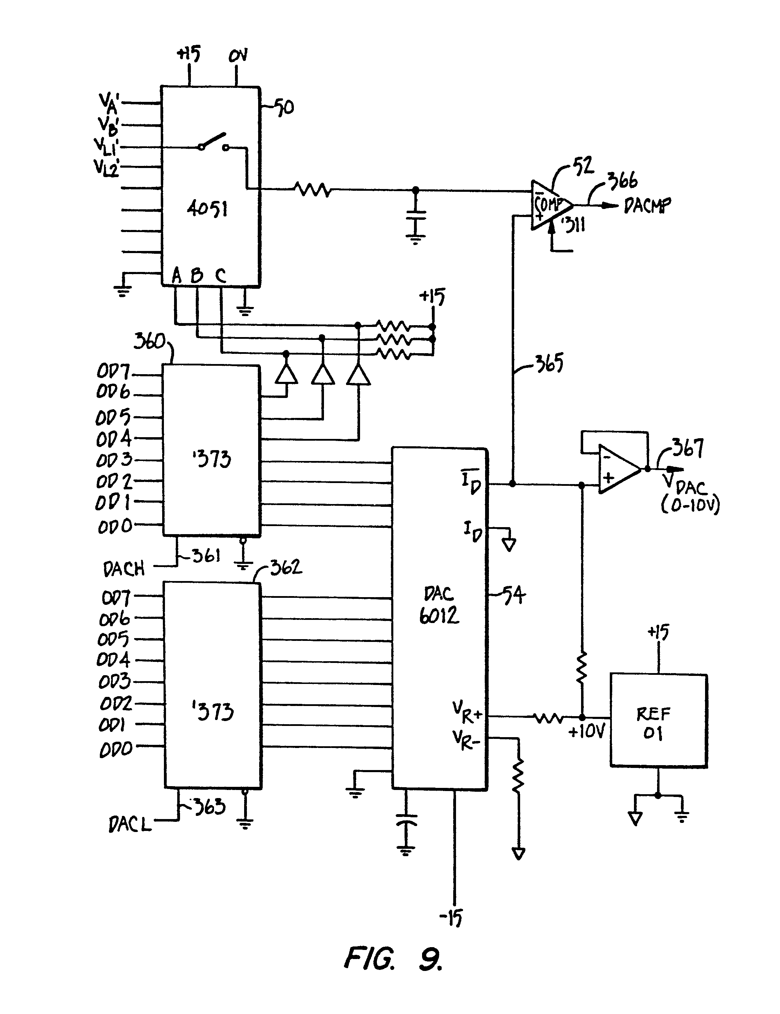

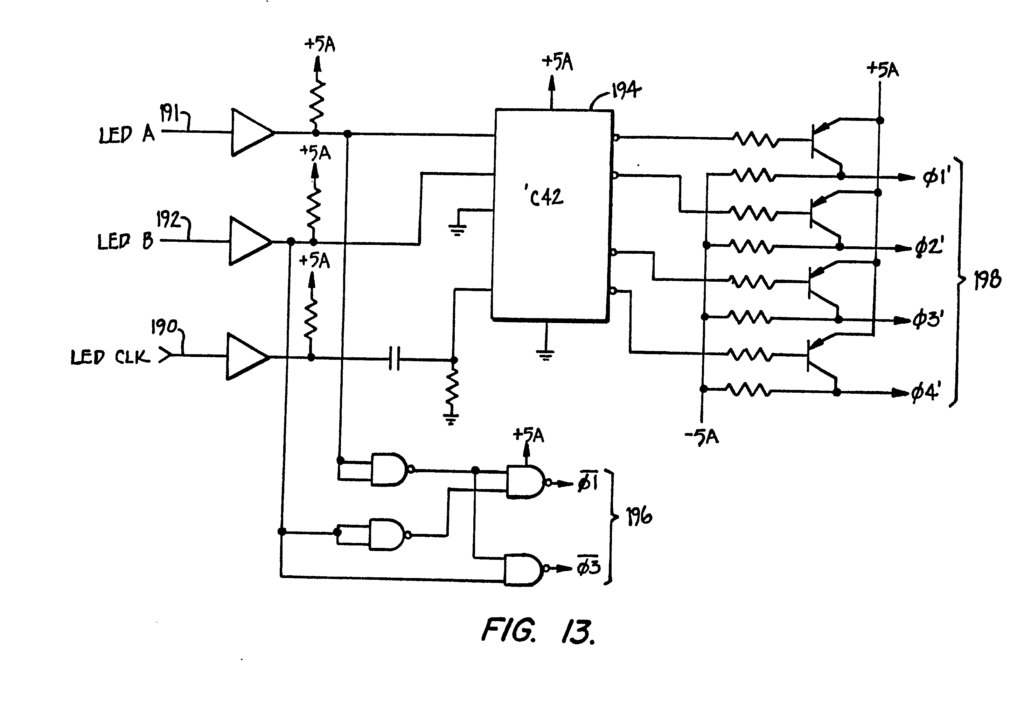

Referring to Fig. 8, a counter used as a divider is illustrated. Referring briefly back to Fig. 3, it will be seen that the microprocessor 100 is provided with a clock running at 2.5 Mhz generally denominated 102. The CPU clock outputs at 102 to a counter 172 (see Fig. 8.) Counter 172 divides signal 102 by the number 171 and outputs to binary counter 173 in order to generate an LED clock frequency of 1.827 kHz, which is unrelated to room light frequencies. Counter 173 outputs signals LED A 191, LED B 192, LED CLK 190. This circuit in cooperation with the circuit of Fig. 13, effects light and detector switching to enable signal phasing.

Having set forth in generality the microprocessor, it will be realized that much of that disclosed is already known in the art. Specifically, complete descriptions of the wiring of this microprocessor can be found in the MCS-8085 Family Users Manual, published October 1979 by Intel Corporation. Those having skill in the art are referred to this publication should question arise about the circuitry thus far described.

Referring briefly back to Fig. 8, LED clock outputs 190, 191, 192 are inputed to the clock divider 194 of Fig. 13. Divider 194 outputs four sequential duty cycle states denominated $\emptyset 1'$ through $\emptyset 4'$. Comple- ments of signals $\emptyset 1$ and $\emptyset 3$ are outputed directly at clock driver outputs 196. It will be noted that all four signals $\emptyset 1'-\emptyset 4'$ are outputed at 198 for timing purposes hereinafter discussed.

Having set forth the timer, the remainder of this disclosure will be broken down into three discrete parts. First, timing for the light emission of the LED's will be discussed. Emphasis will be placed on the fact that the diodes are switched locally.

Second, light reception will be set forth. With respect to the reception, emphasis will be made to the fact that the signal is digitally extracted without any analog treatment whatsoever. The pure digital signal is thereafter processed and utilized to create the light curves herein. Effort is made to eliminate all variables present, including those in the flesh analyzed as well as ambient light noise.

Thirdly, and in view of variant light transmission qualities of human flesh, the light level adjustment circuit of this invention will be traced. It will be pointed out that the adjustment of the emitted light occurs so that the sensor receives an amount appropriate for the amplification circuitry.

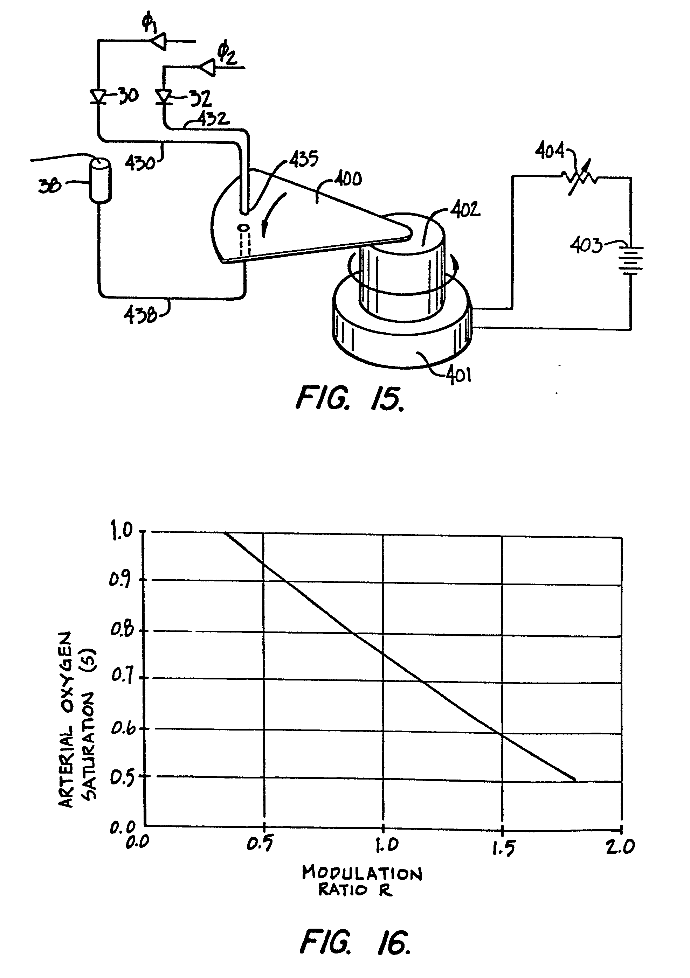

Referring to Fig. 14, and assuming that sufficient voltage is present across leads 301, 302, current of an appropriate level will be emitted to each of the light emitting diodes 31, 33. The diodes here are illustrated schematically across a connector 305 and are shown being switched by respective transistors 307, 309. Specifically, when a negation pulse is received at each of the transistors, the transistors open, voltage appears across the respective diodes 31, 33, and light is emitted.

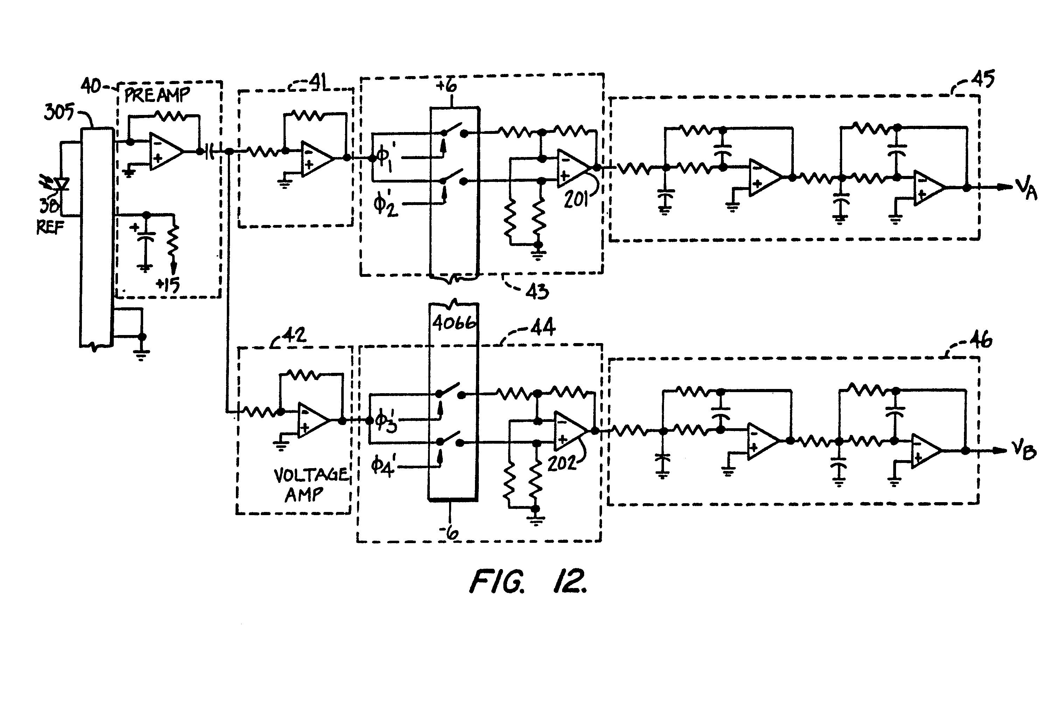

Assuming light is transmitted, it is passed to the flesh of the digit 14 and is thereafter received at the receiving photosensor 38. (See Figs. 2 and 12)

Referring to Fig. 12, photosensor 38 is illustrated. It is coupled across a connector 305. Connector 305 in turn passes its signal through amplifier 40. The signal is then split and passed to voltage amplifiers 41, 42, the amplification here occurring in parallel, allowing differences in gain between red and infrared signal processing. Respective phase detectors 43, 44 are clocked at inputs $\emptyset1'$ - $\emptyset4'$ from the clock circuit of Fig. 13. Remembering that a 1 in 4 duty cycle is here utilized with each of the signals $\emptyset1$, $\emptyset2$, $\emptyset3$, $\emptyset4$ being clock periods, it is seen that the signal is gated. Specifically, and during the $\emptyset1$ time period, negative amplification of the total light signal, including pulsatile component and noise, occurs at amplifier 201 with passage of the resultant signal through the low pass filter 45.

Referring to Figure 14, in the next sequential time period, and due to the signal $\emptyset1$ no longer appearing to close transistor 309, transistor 31 will be shunted to ground. At the same time, during time period $\emptyset2$ gate 43 will open to amplify the positive component received. This component received, however, will have no light emission whatsoever; it instead will represent pure electronic or optical noise. The timing of this circuit will therefore yield on equal bases first light containing the pulsatile component and noise and thereafter just noise. Amplifier 201 amplifies one signal positively and the other signal negatively in equal amounts. It will be seen that integrated over the full four periods of the clock, through amplifier 201 the instrument sees equal components of noise which cancel and unequal components of signal which do not cancel. By the expedient of taking the respective intermittent pulses and passing them through the low pass filter 45, there results a signal out containing valid signal only; noise cancels.

The remaining channel is analogous. Specifically, during time period $\emptyset 3$, noise and light signal are amplified negatively and passed through low pass filter 46. During time period $\emptyset 4$, noise only is positively amplified and cancelled in passage through the low pass filter 46.

The emitted signals $V_A$ and $V_B$ can be described as having two components. The first component is constant. It is that element of light which remains essentially invariant. This signal includes an absorption component because of skin pigment, bone, flesh and veinous blood.

The second component represents that pulsatile inflow of arterial blood.

The ratio of that second component to the first component is what is sought by the instrument. What is sought is the ratio of the arterial and pulsatile component of the blood to that of the total absorbing tissue. The color of the arterial component of the blood produces the differential light transmission that is dependant upon the oxygen saturation of the hemoglobin. The instrument must isolate this component.

Referring to Figure 11, amplification of the signal to an idealized state is illustrated. Specifically, in taking respective signals VA', VB', an offset voltage VOFF is introduced. This signal is a constant voltage which subtracts out part of the constant portion of the received light signal which relates to passage through the nonvariant portions of the flesh. Since it is known that the pusatile component is always very small with respect to the total signal, an improvement on the accuracy of digital conversion can be obtained by this subtraction. It is necessary, however, for the microprocessor program to mathematically reinsert this subtracted voltage prior to processing the signal. This subtraction and amplification occurs at the respective amplifiers 330, 331 with passage of the signals VA' and VB' from the network.

It is important to note that the referred to subtraction does not vary as a function of the detected pulseatile signal. Instead, the referred to subtraction functions only to isolate amplification to the relevant portion of the detected signal only.

With digital to analog conversion of these signals, a combination of the pulsatile component and the remainder of the constant component is then required. This can best be seen through the circuitry of Figure 9.

Referring to Fig. 9, a multiplexor 50 is illustrated. During the analytical operation here shown, this multiplexor samples signals VA' and VB'. Signal is passed to the negative side of comparator 52. Signal for driving the multiplexor passes through lines OD4 - OD6 in the DAC high latch 360. The DAC low latch 362 is thereafter actuated in sequence responsive to enabling signals on enabling line 363. Output occurs to a digital to analog converter 54 on a twelve bit basis. Division to one part in 4096 occurs.

Typically, the signal is compared in halves. Output of DAC 54 occurs over lead 365 to comparator 52. The DAC output 366 is passed to the microprocessor. Depending upon whether a high or low signal is received, stepping of the twelve bit DAC 54 occurs in halves, enabling the twelve bit division to occur rapidly. Consequently, the output level of the voltage of the receiving photosensor is rapidly determined with the result that the pulsatile component can be rapidly followed.

This process is repeated for both signals VA' and VB' at a rate that allows the microprocessor to faithfully track both signals.

Having set forth the light reception circuitry of this invention, attention will now be directed to the level of light adjustment.

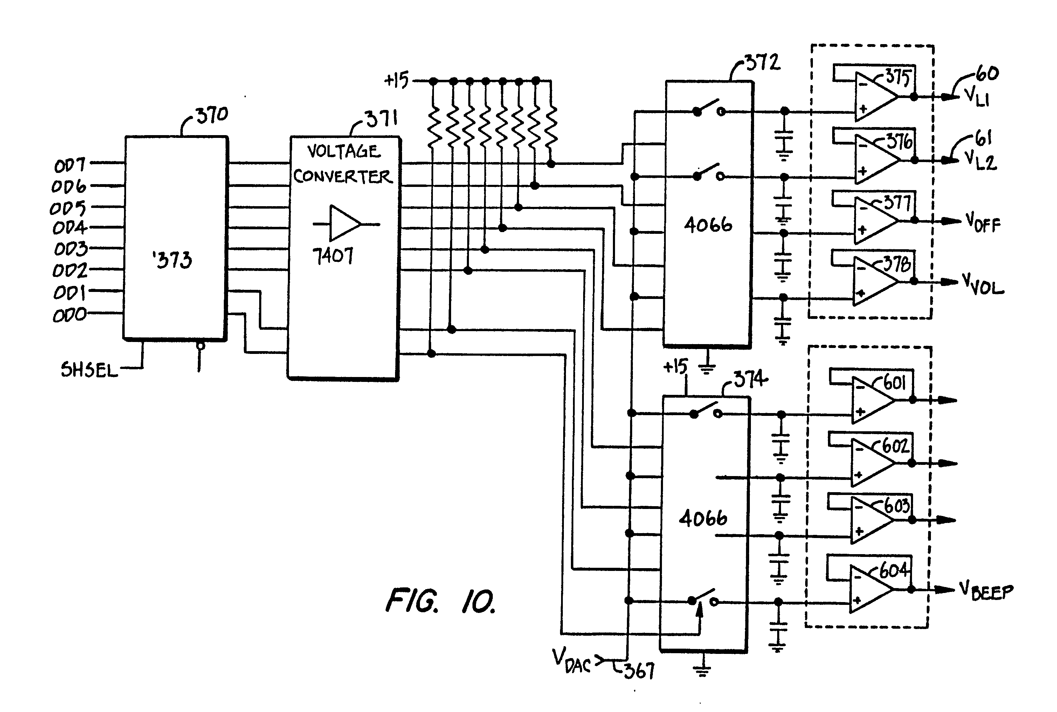

It will be remembered that each of the patients, due to flesh, skin pigment, skin thickness, bone, veinous blood present and other invariants, will present his own factor of constant light transmission at both wavelengths. This being the case, it is necessary to adjust the level of current applied to the light sources 31, 33. This is done through the DAC circuit of Fig. 9 and the sample hold circuit of Fig. 10.

The sampling of the light signals by the microprocessor was described above. In the case where the signals are not within the useful range of the conversion circuitry, the light level must be adjusted up or down as required to restore the signal level to the voltage range acceptable to the analog to digital conversion. Referring to Figure 9, the program will output a code corresponding to the desired voltage level through its data bus into latches 362 and 360, setting the DAC 54 output to a voltage corresponding to desired LED current. Note that this is only done during a time period when the DAC is not used for input conversion. The program will then output using the same bus a bit corresponding to the selected LED into latch 370 of Fig. 10. This bit, or selection signal, is converted to a compatable voltage by voltage converter 371 and applied to one of eight analog switches 372 and 374. These have the effect of applying the voltage from the DAC, corresponding to the desired LED current level, to a storage capacitor which will latch this voltage after the input has been removed. This voltage is buffered by amplifiers 375 and 376 and applied to the LED circuitry. Thus, dependant upon the intensity of the signal received by the photosensor, the respective light emitting diodes can be driven with greater or lesser voltage to produce the optimum voltage output.

It is noted that only two of the available eight channels of this sample hold circuitry are required to adjust the LED intensities. The remaining channels provide a general purpose analog output from the microprocessor for a variety of unrelated functions. The output of amplifier 377 provides the fixed offset for the offset amplifiers described above; the output of amplifier 378 provides a volume control for the alarm; outputs of amplifiers 601, 602, and 603 provide external outputs for an optional chart recorder; and the output of amplifier 604 provides a control for the pitch of the alarm.

### THEORY OF OPERATION

The method of operations involves taking measurements of light transmission in tissue at two distinct wavelengths (red and infrared) at two arbitrary points in time, these points in time being but a small fraction of the time for a complete pulse. The wave form of a pulse of blood in the human flesh is digitally plotted. By considering the change in the transmission of light due to inflowing arterial blood, a measurement is made.

Regarding this transmission, as blood flows in, light is absorbed. Consequently the resident detector of light, photosensor 38, sees less light. Thus, it is the drop in light received at the photosensor that indicates the pulsatile component.

Assuming that the ambient transmission (approximately 99% of the signal) is represented by the letter I and the change in transmission during the pulse is defined by the letter $\Delta I$ then the equation for representing the change in transmission relative to the unchanging matter in the flesh to be integrated is represented by the equation:

(1)

$$\frac{\Delta I}{I} \ \alpha \ \Delta M$$

where $\Delta M$ is the change in material in the flesh during the pulse.

Interposing a constant to produce an equation yields the form:

(2)

$$\frac{\Delta I}{I} = K \Delta M$$

where K is a constant of proportionality in the resultant equation.

Realizing that the change in mass is composed of blood whose optical absorption is larger than the tissue and that this blood includes two forms of hemoglobin: oxyhemoglobin (hemoglobin with appended oxygen) and reduced hemoglobin (hemoglobin without oxygen), this equation can be expanded for two variants of matter thus:

(3)

$$\frac{\Delta I}{I} = K_A \Delta M_A + K_B \Delta M_B$$

where $K_A$ is a constant for oxyhemoglobin; $\Delta M_A$ is the amount of matter due to the influx of oxyhemoglobin; $K_B$ is a constant for reduced hemoglobin and $\Delta M_B$ is the change in reduced hemoglobin.

It will be remembered, that we are conducting our examination at two discrete wavelengths. This being the case, the above relation can be expanded to include the applicable constants at each wavelength thus:

(4a)

$$\frac{\Delta I}{I} \ \bigg|_{\lambda 1} = K_{A1} \ \Delta M_A + K_{B1} \ \Delta M_B$$

(4b)

$$\frac{\Delta I}{I} \ \bigg|_{\lambda 2} = K_{A2} \ \Delta M_A + K_{B2} \ \Delta M_B$$

where $K_{A1}$ and $K_{B1}$ are the respective oxyhemoglobin and reduced hemoglobin constants at a first wavelength $\lambda 1$ (say the red wavelength) and $K_{A2}$ and $K_{B2}$ are the constants at a second wavelength $\lambda 2$;

It will be appreciated that each of the constants having the form $K_{xy}$ is a constant that relates the relation of the change of light absorption to the total light absorption for a particular color and change of matter due to pulsatile flow.

Realizing that we are after the fraction S (saturation) of oxyhemoglobin to total hemoglobin then we know that:

(5a)

$$\Delta M_A = S \, \Delta M$$

(5b)

$$\Delta M_B = (1-S)\Delta M \text{ where } \Delta M = \Delta M_A + \Delta M_B \quad (6)$$

where S equals the saturation and (1-S) equals the fractional presence of the reduced hemoglobin. Placing this into the previous equation yields the results:

(6a)

$$\left. \frac{\Delta I}{I} \right|_{\lambda 1} = K_{A1} \, S \, \Delta M + K_{B1} \, (1-S) \, \Delta M$$

(6b)

$$\left. \frac{\Delta I}{I} \right|_{\lambda 2} = K_{A2} \, S \, \Delta M + K_{B2} \, (1-S) \, \Delta M$$

It can be seen from the above equations that one saturation is determined, solution for blood perfusion ($\Delta M$) is trivial.

At this juncture, we surprisingly define a ratio related to the light transmission at two different wavelengths. In defining this ratio, the reader will realize that we avoid manipulation in accordance with logarithmic proportionality. Specifically, we define the ratio between light transmitted and received at the wavelength $\lambda 1$ and at the wavelength $\lambda 2$ as follows:

(7)

$$\theta \propto \frac{\frac{W\uparrow}{\uparrow} \sqrt{g^1}}{\frac{W\uparrow}{\uparrow} \sqrt{g^2}}$$

Substituting the values of change of light·
absorption over total light transmission yields:

(8)

$$R = \frac{K_{A1} S + K_{B1} - S K_{B1}}{K_{A2} S + K_{B2} - S K_{B2}}$$

Likewise, substituting for S yields:

(9)

$$S = \frac{K_{B1} - RK_{B2}}{R(K_{A2} - K_{B2}) - (K_{A1} - K_{B1})}$$

Thus, it can be seen that a relationship
exists for both the ratio R and the saturation S.

Those in the medical arts will realize that
the numbers sought to be determined electronically by
the absorption of light, are also capable of laboratory
tests. Specifically, there are a number of laboratory
protocols and tests whose accepted results yields
saturation. This being the case, a procedure for the
calibration of all instruments becomes immediately
apparent.

Specifically, by taking laboratory arterial
oxygen saturations from individuals at differing satur-
ations $S_1$, $S_2$, $S_3$, and $S_4$, we can measure specific
transmission ratios $R_1$, $R_2$, $R_3$, and $R_4$. To reliably
obtain these ratios $R_i$ the present instrument itself is
used, in particular the portion of the device related
to obtaining reliable measurements at hand herein. We
thereafter can make an initial guess as to coefficients
for both oxyhemoglobin and reduced hemoglobin.

Taking one of the aforementioned constants
$K_{A1}$, this constant can be broken down into two discrete components. First, one component can come from a previously determined value and be denominated $C_{A1}$. Secondly, and for each instrument, this constant will of necessity change. This change will be due to the conditions of observation, individual instrument electronics and the like. This value can be express $\Delta C_{A1}$. Each of the four constants in the above equation can likewise be expanded in the same way.

(10)

$$(C_{A2} + \Delta C_{A2})(S_i R_i) + (C_{B2} + \Delta C_{B2})(R_i - S_i R_i) =$$
$$(C_{A1} + \Delta C_{A1})((S_i) + (C_{B1} + \Delta C_{B1})(1-S_i)$$

where i is an index related to at least four measured saturations and transmission ratios.

Those skilled in math and instrument calculation can now see that the $\Delta C$ quantities in all states and wavelengths can be simultaneously solved provided that four independent saturations are utilized. Therefore, a set of constants is attained, which constants can be utilized for programming individually produced instruments at all values of S.

The aforegoing relations can be alternatively determined. We have found that the relation of R (the ratio of transmission) to S (saturation of the hemoglobin with oxygen) is capable of simple curve fitting. Specifically, for at least human beings a constant and predictable curve of S with respect to R results. A plot of this curve is shown as figure 16. By utilizing this relationship in a look-up table, one may quickly compute the saturation of a patient.

Note that in our device, unlike in the prior art, a light source of isobestic wavelength is not used, nor is apparatus for taking logarithms necessary (see equation 7).

The reader will realize that the disclosed pulse oximeter or plethysmograph is targeted for use typically on a human digit. It should be realized that the disclosed pulse oximeter works equally well on any number of cutaneous locations. Idealized and a preferred use of the extremely small and local sensor of this invention is on the scalp of children being born. Avoidance of oxygen poor conditions during birth resulting in cerebral palsy is contemplated. Likewise, any other cutaneous location will suffice, e.g. the nasal septum.

## INSTRUMENT CALIBRATION

Referring to Fig. 15, an apparatus for the on-site calibration of this instrument is disclosed. Specifically, the device of Fig. 15 allows the adjustment of the ratios R and substitutes a rotating filter 400 for the pulsatile component of blood in a human being.

Viewing the apparatus of Fig. 15, a motor 401 having a conventional power source 403 here schematically shown as a battery is illustrated. A varible resistor 404 drives the motor at varying rates of rotation. Typically the rates of rotation are selected so that human pulse is emulated. It will be apparent that although only one filter element is shown, multiple elements could be used with multiple variations.

The red light source 30 and infrared light source 32 are addressed to respective light pipes 430 and 432. These respective light pipes define light paths and pass light to a position 435 immediately overlying the rotating filter 400. At the same time, the photosensor 38 is coupled to a second light pipe 438. Light pipe 438 thus receives first unattenuated light and thereafter light attenuated by the filter 400.

Filter 400 is rotated on motor 401 by rotation of the rotor 402. In such rotation, the filter passes between the respective light emulating the pulsatile signal.

Since the pulsatile component of blood flow is represented by the increasing amount of absorption, it has been found that the simple device of rotating filter 400 emulates the pulsatile component of the finger. Naturally, those skilled in the art will realize that tailoring of the filter 400 can be accomplished. For example, by putting a graded neutral density filter across the surface of the filter 400, and having the filter sensitive to the red and infrared components here measured, a close approximation to the pulsatile reaction of the finger can be accomplished. Likewise color can be omitted entirely, the limitation being that the color of the light sources will not be verified.

Secondly, for neutral density filters the same across the red and infrared light patterns, the respective ratio R will be constant. Therefore the rotating filter can be utilized for the calibration of the ratio R computed. The reader will also see that the filter density may vary radially so that the position of the light pipe will produce varying R's.

Referring to Fig. 16 a plot of the ratio R with respect to experimentally determined blood oxygen saturations S is shown. Those skilled in the art will realize by the art of curve fitting the respective constants necessary for correlating a given ratio R to a given saturation S can be easily obtained.

The reader will realize that red and infrared have been preferably used. Any two colors which produce a non-redundant set of R and S can be used--these colors, however, are preferred.

CLAIMS:

1.      A pulse oximeter including a light emitting means for directing light into the flesh of a patient characterised in that the means includes first and second sources for emitting sequential light pulses in the red and infrared ranges;  at least one sensor sensitive to each of said light sources arranged in use to have a light path through the flesh of the patient from the first and second sources;  each light source having duty cycle of or less than one part in two with respect to time, the sensor in use viewing light transmission from each light source during a first part of the duty cycle of each light source and viewing ambient light during a second part of the duty cycle of each light source;  the sensor outputting signals in use to an amplifier, the amplifier through putting the signal from the sensor during the first part of the duty cycle and through putting a signal corresponding to ambient light during a second part of said duty cycle;  and means for subtracting one of the amplified signals from the other of the amplified signals so that a signal or signals due to ambient light is or are substracted from the sensed light signal.

2.      An oximeter according to Claim 1 and wherein the amplifier includes a positive input and a negative input and further including means for switching the first part of the duty cycle of each light source to one of said amplifier inputs and the second part of said duty cycle of each light source to the input of opposite sign of said amplifier.

3.      A pulse oximeter comprising at least one light source for outputting light of a preselected wavelength into the flesh of a patient; at least one light sensor addressed to the flesh of said human adjacent the input of said light source having an indirect path through the flesh of said human from said light source characterised by;means for continually monitoring intensity of light received by said light sensor to detect increasing absorption of light in a first time period due to the arterial inflow of blood to said flesh and decreasing absorption of light in a second time period due the the arterial outflow of blood; and means for adjusting the light intensity output of said light source during one of said light periods only to maintain a predetermined range of light intensity at said light sensor during said arterial flow only, whereby optimum tuning of said monitoring means occurs.

4. An oximeter according to Claim 3 wherein said means for adjusting the light level of said light source during one of said light periods is synchronized to the second time period, so that adjustment of the level of the light source occurs during the arterial outflow of blood.

5. An oximeter according to claim 3 or 4 including two light sources for outputting light of differing preselected wavelengths into the flesh of a patient and wherein the intensities of both the light sources are adjusted in the second time period during the arterial outflow of blood.

6. A pulse oximeter comprising a light emitting means for directing light into the flesh of a patient characterised in that the means includes first and second light sources respectively in the red and infrared ranges; and by at least one light sensor addressed to the flesh of said patient adjacent said light sources to receive light passing through the flesh from said light sources; the light sensor for sensing light from the light sources and outputting sequentially a first signal from the first light source and a second signal from the second light source, each light source having alternate sequential duty cycles of less than one part in two parts of time, so that said light sensor first samples the light from the first light source passing through the flesh of said human, thereafter the light sensor secondly samples ambient light passing through the flesh, thirdly samples the light from the second light source passing through the flesh and fourthly samples ambient light passing through the flesh; a comparison circuit connected to the output of the sensor, this comparison circuit comparing the output of a digital to analog converter to the light received from the light source, means for producing a ratio of the varient portions of the light signals to the non-varient portions of the light signal.

7.        An oximeter according to claim 6 further including first and second amplifiers connected to the output of said comparison circuit, the amplifiers receiving an offset signal which constitutes an invarient portion of the output of the comparison circuit and negatively amplifying the offset signal and the first and second amplifiers receiving the first and second light signals respectively, so that the outputs of said first and second amplifiers constitute the varient portions of light signals, the outputs of the first and second amplifiers omitting a substantial portion of the invarient portions.

8.        A pulse oximeter for determining arterial oxygen saturation and indication volume of blood flow in a patient, said oximeter including light emitting means characterised by the means, comprising first and 'second light emitting sources for emitting sequential light pulses in the red and infrared into the flesh of a patient; and by a sensor sensitive to each of the light sources having an indirect light path through the flesh of said human from said first and second light sources; said sensor sequentially outputting signals to an amplifier from each of said light sources; means for digitally tracking the light absorption; means for dividing the change of light transmission due to the pulsatile component of blood flow with respect to the total light transmission to determine a quotient of light absorption for each optical wavelength to determine blood flow; means for making a ratio related directly to the respective quotients of light transmission at each said frequency and means for fitting the ratios of light transmission to experimentally determined saturations at said ratio to enable the optical determination of saturation.

9. An oximeter according to claim 2 or 8 and wherein the light sources have a duty cycle of one part in four.

10. An oximeter according to claim 8 or 9, wherein the means for digitally tracking the light transmission includes for each said frequency a first positive input including the transmitted light signal and a second time input subtracting out ambient lighting from the transmitted light signal preferably substantially only the variant portion of the light transmission pulse being amplified.

11. The oximeter of claim 1 including a calibrating instrument.

12. A calibrating instrument for an oximeter comprising at least one light path arranged to communicate at a light receiving end with the light sources of the oximeter and a light emitting end to a spacial interval overlying the path of said filter; at least a second light path communicated from the outlet of said first light pipe to transmit light to the photosensor of said oximeter; means for passing a filter having a absorption component through said interval between said light pipes to emulate the pulse.

1/10

FIG. 1.

FIG. 8.

FIG. 2.

FIG. 3.

FIG. II.

FIG. 4.

FIG. 5.

5/10

FIG. 6.

FIG. 7.

FIG. 14.

FIG. 9.

FIG. 10.

7/10

0102816

FIG. 12.

FIG. 13.

FIG. 15.

FIG. 16.